# EUROPEAN PATENT APPLICATION

(11) **EP 4 283 630 A1**
(43) Date of publication of application: **29.11.2023**
(21) Application number: 22174882.5
(22) Date of filing: 23.05.2022
(51) Int. Cl.: G16H 40/63, G16H 50/20

(54) **LIGHT EMITTING HAIR GROWTH MANAGEMENT DEVICE COMPANION APPLICATION**

(71) Applicant: Braun GmbH, 61476 Kronberg im Taunus (DE)
(72) Inventor: KAMYSHANSKA, Hanna, 61476 Kronberg (DE); RIETZLER, Miriam, 61476 Kronberg (DE); FRIEDRICH, Anette, 61476 Kronberg (DE); BIELFELDT, Uwe, 61476 Kronberg (DE)
(74) Representative: P&G Patent Germany

(57) **Abstract**

The invention provides techniques for estimating a skin coverage in a usage session of a light-emitting hair growth management device. Sensor data from one or more sensors of the light-emitting hair growth management device is used by a rules-based algorithm and/or machine learning algorithm to estimate the coverage of a target body part or parts achieved in the usage session. This information is fed back to a user of the hair growth management device via a graphical user interface . In another aspect the invention provides a scheduling application that is able to dynamically adjust a usage plan comprising one or more usage sessions of a light-emitting hair growth management device based on information about the level of hair growth management achieved in a given usage session.

## Description

### FIELD OF THE INVENTION

The invention relates generally to the monitoring of the usage of light-emitting hair growth management devices. Specifically, the invention relates to the calculation of a skin coverage achieved during one or more usage sessions and/or to the dynamic scheduling of one or more usage sessions.

### BACKGROUND

Light-emitting hair growth management devices are becoming increasingly popular, particularly in settings such as a salon or in the home. In such settings it is common that the operator of the device has not received an intensive training course on the use of the device. That is, the operator is reasonably skilled in the operation of the device but is not an expert. As a result, the operator may not be able to objectively judge the effectiveness of a given usage of the device, which could lead to suboptimal results and user dissatisfaction. This is particularly because some time usually passes between a usage session of the hair growth management device and the effects of the device to be visible, meaning that unlike e.g. a shaver or razor, the operator cannot receive immediate (visual) feedback on the effectiveness of the usage session.

A characteristic of light-based hair growth management techniques is that they are most effective when used according to a recommended schedule. An operator can be provided with a recommended schedule but in practice they may not follow this correctly, e.g. due to an inability to use the device when scheduled, such as when a customer misses or reschedules a salon appointment, or because the operator forgets to use the device when scheduled. Recommended schedules are often provided in a static manner, e.g. as instructions in a leaflet or pamphlet provided with the device. A static format like this cannot take account of the actual usage of the device and adjust the schedule accordingly, which may lead to suboptimal results and user dissatisfaction.

It is therefore desirable to provide a mechanism for enabling an untrained person to objectively judge the effectiveness of a given usage of a light-emitting hair growth management device. Preferably, this mechanism would be user-friendly and easy to understand for an untrained person.

It is also desirable to provide a mechanism for enabling a dynamic schedule for usage of the light-emitting hair growth management device to be generated, where this usage schedule is adjusted based on actual use of the hair growth management device.

### SUMMARY OF THE INVENTION

The invention has two main aspects. The first aspect relates to the estimation of a skin coverage achieved during a usage session of a hair growth management device. The skin coverage is automatically estimated using sensor data collected by one or more sensors of the hair growth management device during the usage session. The skin coverage estimate can be provided during the usage session (i.e. in real time, or near real time), or it can be provided after the usage session is complete. This objective and automatic assessment allows an operator of the hair growth management device to easily understand how effective a given usage was, which can lead to improved user satisfaction.

The second aspect relates to a dynamic scheduling of usage sessions in a usage plan of a hair growth management device. A usage plan including one or more usage sessions is generated for a person who is to be the subject of the usage session. The usage plan may include two or more usage sessions, e.g. 2, 5, 10, 20 usage sessions. The usage plan is presented by a scheduling application executing on an electronic device, e.g. in the form of a calendar in a calendar application. After each usage of the hair growth management device, the operator or user enters information relating to the hair reduction effects actually observed, or alternatively this information is collected automatically by processing image(s) of the body part that is the subject of the usage session. The scheduling application is configured to adjust the usage plan based on the information supplied by the user or collected automatically. In this way a dynamic usage plan is created that adjusts scheduled usage sessions according to the actual usage of the device. This can lead to improved user satisfaction as the user is better able to understand how to use the device effectively.

The first aspect can be implemented by a computer-implemented method for monitoring the usage of a light-emitting hair growth management device in a usage session of the hair growth management device, the method comprising: receiving, by a processor, sensor data from one or more sensors of the hair growth management device, the sensor data gathered during the usage session; performing, by the processor, a calculation of a skin coverage achieved in the usage session using the sensor data; and displaying, on a display of a user device or on a display of the hair growth management device, information relating to the calculated skin coverage

The first aspect can also be implemented by a hair growth management system comprising a hair growth management device, a processor and a display, the hair growth management system configured to perform the method of the first aspect.

The second aspect can be implemented by a computer-implemented method for adjusting a usage plan for a light-emitting hair growth management device, the method comprising: providing, in a scheduling application of an electronic device, a usage plan comprising one or more planned usage events, the or each planned usage event having a corresponding date associated with said or each planned usage event; obtaining, by the electronic device, an indication of a hair density of hair present on a body part associated with the usage plan; and adjusting, by the electronic device, a date of at least one of the one or more planned usage events based on the indication.

The second aspect can also be implemented by a non-transitory computer-readable medium storing instructions thereon which, when executed by a processor of an electronic device, cause the electronic device to: provide, in a scheduling application of an electronic device, a usage plan comprising one or more planned usage events, the or each planned usage event having a corresponding date associated with said or each planned usage event; obtain an indication of a hair density of hair present on a body part associated with the usage plan; and adjust, by the electronic device, a date of at least one of the one or more planned usage events based on the indication.

Further preferred features of the first and second aspects are set out in the appended dependent claims and/or detailed description.

### BRIEF DESCRIPTION OF THE DRAWINGS

Embodiments of the present invention are described below, by way of example only, with reference to the accompanying drawings, in which:
Figure 1 illustrates in schematic form a light-emitting hair growth management device suitable for use with embodiments of the invention;
Figure 2 illustrates in schematic form a system that is suitable for implementing embodiments of the invention;
Figure 3 is a flow chart depicting a method for monitoring usage of a hair growth management device according to an embodiment; and
Figure 4 is a flow chart depicting a method for automatically adjusting a usage plan for a hair growth management device based on user feedback according to an embodiment.

### DETAILED DESCRIPTION

As used herein, the term 'user' can be the person that a light-emitting hair growth management device is being used on or an operator of the device, who may be a different person to the person that the device is being used on.

The term 'skin coverage' refers to a parameter that indicates a proportion of an area of skin that is exposed to one or more pulses of light from the hair growth management device during a usage session. The skin coverage can be calculated from data provided by device sensors as described below. The skin coverage can be expressed as a percentage or fraction of a target body part, for example. As the hair growth management effect of the device is derived from exposing skin to light pulses, it follows that skin coverage is a relevant parameter when assessing the effectiveness of a usage session. Effectiveness here refers to the level of hair growth management that results from the usage session.

The first aspect of the invention provides techniques for estimating a skin coverage in a usage session of a light-emitting hair growth management device. Sensor data from one or more sensors of the light-emitting hair growth management device is used by a rules-based algorithm and/or machine learning algorithm to estimate the coverage of a target body part or parts achieved in the usage session. This information is fed back to a user of the hair growth management device via a graphical user interface. The graphical user interface can be displayed on a display of a user device or on a display of the hair growth management device. Further detail on this first aspect is provided directly below.

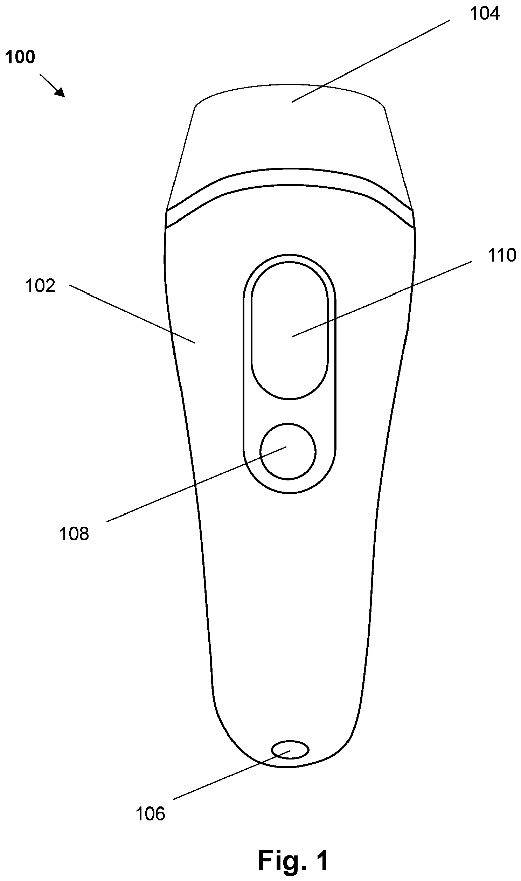

Fig. 1 illustrates a light-emitting hair growth management device 100 in schematic form. Device 100 could be a photo-epilator of a type known per se in the art, for example. The construction and operating principles of a photo-epilator are known and so these are not described in detail here in the interests of brevity.

Device 100 includes a housing 102 and a head 104 that includes, encloses or is otherwise associated with, a light-emitting component (not shown). The light-emitting component is typically a flashlamp housed in an optical window that is transparent at least to light of wavelengths known to have hair growth management effects.

Head 104 is preferably removably attached to housing 102 to enable the operator to attach different heads to housing 102. Each head may be particularly suited for use on a specific body part or parts, for example.

A power supply 106 is also present. This may comprise a battery and/or a port that enables a wired electrical coupling with an electrical socket. Inductive charging techniques can also be used. Power supply 106 provides electrical power for operating various components of device 100, including the light-emitting component, processor and transmitter/transceiver, for example.

A power button 108 is provided in housing 102. This enables an operator of device 100 to turn device 100 on and off. Device 100 may be configured to automatically turn off and/or enter a power saving sleep mode after a predetermined period of inactivity, e.g. 5 minutes, to avoid power wastage. Power button 108 can be a push button or slider coupled to a switch, for example.

An activation button 110 (or 'trigger') is also provided in housing 102 to enable an operator of device 100 to trigger a pulse of light (often termed a 'flash') from the light-emitting component. Activation button 110 can be a push button coupled to a switch, for example. Activation button 110 can be coupled to a skin sensor (not shown) that enables device 100 to determine whether head 104 is currently proximate skin. Such skin sensors are known in the art. Activation button 110 may be disabled, e.g. by ignoring input from activation button 110, in the case where it is determined that head 104 is not currently proximate skin, for safety reasons.

Device 100 preferably includes a skin tone sensor (not shown) that is capable of automatically determining the tone of skin. This may be the same sensor as the skin sensor described in the immediately preceding paragraph, or a different sensor. Skin tone sensors are known per se.

Device 100 can also include a parameter setting mechanism (not shown) for setting operating parameters of device 100. This mechanism can include one or more sliders, wheels, etc., where each slider or wheel corresponds to a particular parameter. Parameters can include: a pulse duration, a pulse fluence, a skin tone (e.g. according to the Fitzpatrick skin type classification), a hair colour, a body part, and the like.

While physical buttons, sliders, wheels, etc. have been described above, it will be appreciated that any of these activation or selection components can be implemented digitally, e.g. via a user input device coupled to a display showing an appropriately configured user interface.

Device 100 also includes a processor (not shown) that may be a microcontroller, for example. The processor is configured to control operation of device 100 as described herein. The processor is communicatively coupled to a memory (also not shown) that is capable of storing data such as sensor data as described below.

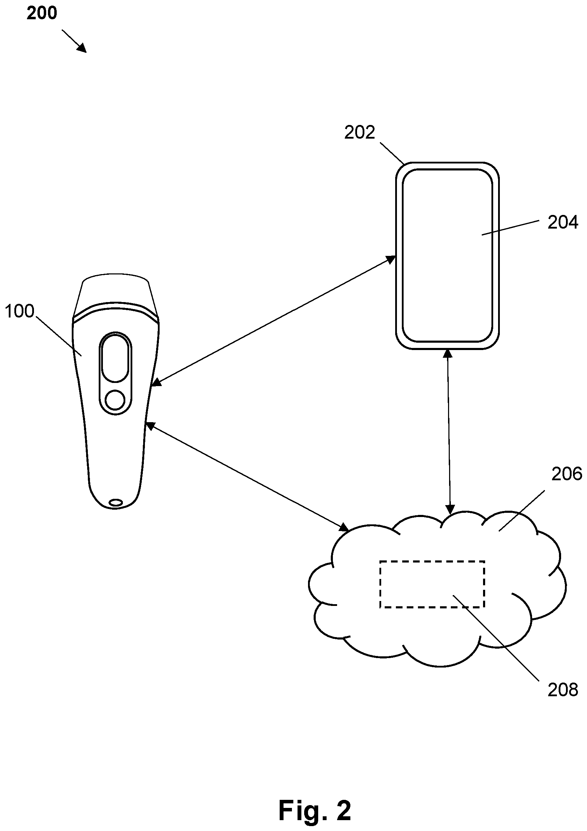

Device 100 additionally includes a transmitter/transceiver (not shown) that enables device 100 to communicate wirelessly with other devices (see Fig. 2). The transmitter/transceiver can be a Bluetooth transmitter/transceiver, preferably a Bluetooth Low Energy (BLE) transmitter/transceiver, a WiFi transmitter/transceiver, a near-field communication (NFC) module, etc. In some cases the transmitter/transceiver can support multiple distinct communication technologies, e.g. both Bluetooth and WiFi. In these cases multiple antennae may be present.

Device 100 further includes one or more sensors (not shown). The sensor(s) function to gather information about the operation of device 100 as relating specifically to the usage of device 100. The one or more sensors can be any one or more of the following types of sensor:
**A)** A flash counter that is configured to count the number of flashes emitted by device 100. The flash counter can use a button press sensor (not shown) that provides a signal each time a button that triggers a flash (e.g. button 110) is pressed. The flash counter may store a flash count in the memory of device 100, for example. The flash count may be reset to zero each time device 100 is switched off, and/or a period of inactivity greater than a predetermined time is detected, for example. The flash count may additionally or alternatively be reset to zero after a skin contact sensor (not shown) determines that device 100 is no longer in contact with skin, or has not been in contact with skin for a time greater than a predetermined time. User input, e.g. to indicate completion of a usage session, could additionally or alternatively be used to trigger a reset of the flash counter to zero.

The flash counter may be configured to store a time at which a flash occurred. This can advantageously enhance the flash count data as it is then possible to determine both a number of flashes and time-based parameters such as a flash frequency, a time between adjacent flashes, and/or averages of these parameters.

Counting the number of flashes enables an objective measure of the skin coverage achieved in the corresponding device usage session to be made, particularly when combined with knowledge of the body part(s) that were exposed to light during the usage session. This information can be fed back to a user by displaying a skin coverage on a display of a user device. Further information on this is provided later in this specification.

Advantageously, the processing resources consumed for performing the counting are relatively low. This type of processing can therefore be performed in real time by a relatively low powered electronic device, such as a user device (e.g. a smartphone, tablet, etc.) In some cases device 100 itself can perform the flash count, with only summary information such as the total number of flashes being transmitted from device 100 to user device 202. This further enhances the ability of the invention to provide a skin coverage estimate in real time.

**B)** An accelerometer and/or gyroscope. (If both are present this may be referred to as an inertial measurement unit, IMU). These sensors can respectively detect and quantify linear and rotational movement of device 100. In particular, accelerometers and gyroscopes generate time series data that enables linear or rotational acceleration to be calculated as a function of time. Use of such sensors can be referred to as tracking the motion of device 100.

By tracking the motion of device 100, an objective estimate of the device's skin coverage can be made. The skin coverage estimate is preferably performed using a trained machine learning model that accepts accelerometer and/or gyroscope data as input and outputs a skin coverage value.

**C)** A skin contact sensor. The skin contact sensor can detect when device 100, particularly head 104, is in contact with skin. The skin contact sensor can provide this data as a function of time, e.g. time series data indicating those moments during the usage session where the device 100 is in contact with skin. Derived quantities such as the fraction of the total usage session that device 100 was in contact with skin can also be calculated.

**D)** A barometric sensor. Data from the barometric sensor is usable to determine a height of device 100 as a function of time. This information can be used to track the motion of device 100 and provide an objective estimate of the skin coverage in a given usage session.

Device 100 can be configured to stream the accelerometer data and/or gyroscope data and/or barometric sensor data and/or skin contact data continuously or quasi-continuously to another electronic device as a data stream. By quasi-continuously, it is meant that data may be buffered for some time by device 100 and then transmitted once the buffer (e.g. the memory of device 100) has reached a predefined fullness. The predefined fullness is preferably less than the total capacity of the buffer.

In a preferred embodiment, device 100 incorporates at least the sensors discussed above under A. In a more preferred embodiment, device 100 incorporates all of the sensors discussed above under A and B, and even more preferably also the sensor discussed under C and optionally also D. The combination of data from all of these sensors can advantageously lead to a calculated skin coverage that is highly accurate.

Device 100 can include a display (not shown) which may also double as a user input device, e.g. a touchscreen. The display can be used to display a skin coverage calculated as described herein. The calculation of the skin coverage can be performed by the processor or device 100 or it can be performed by another processor that is not part of device 100 and transmitted to device 100 for display. If present, the display of device 100 can additionally or alternatively be used to display a schedule of the type discussed later in this specification.

Device 100 can include an image capture module (not shown) such as a camera. The image capture module is preferably located such that it can readily capture image(s) of the body part(s) that is/are the subject of a usage session. Images captured in this way can be processed to estimate a hair density. More information on this is provided later in this specification.

Referring now to Fig. 2, a system 200 is shown that is suitable for implementing embodiments of the invention. System 200 includes device 100, a user device 202 and optionally also Cloud 206.

In Fig. 2 arrows are used to show communication paths between the various components of system 200. As can be seen, device 100 is communicatively coupled to user device 202 via the transmitter/transceiver discussed above, e.g. via a WiFi or Bluetooth connection. Although two-way communication is shown in Fig. 2, this is not limiting on the invention as in some embodiments device 100 makes use of a transmitter that provides one-way communication from device 100 to user device 202.

User device 202 can be any electronic device capable of performing the functions attributed to user device 202 as described herein. User device 202 could be, for example, a smartphone, a tablet, a laptop, a wearable electronic device like a smart watch, a desktop computer, etc. User device 202 includes a display 204 of the type known in the art per se. User device 202 also includes one or more user input components (not shown) such as a touchscreen, keyboard, etc. User device 202 further includes a processor and memory (not shown).

It is also contemplated that the function(s) of user device 202 as discussed herein could alternatively be carried out by device 100. Thus, the invention is not restricted to system 200 and other embodiments exist in which user device 202 is omitted. In these embodiments, all functions discussed herein in connection with user device 202 are performed instead by device 100 and/or Cloud 206 (if present).

Cloud 206 is a cloud computing environment of the type known in the art. Briefly, Cloud 206 provides processing resources remote from device 100 that can be tasked with particular activities. It should be understood that the processing capabilities of cloud 206, e.g. processor speed, number of processors available, amount of memory available etc., tend to significantly exceed those of user device 202 and device 100. Cloud 206 is therefore more suited to performing more processor-intensive operations than user device 202.

Device 100 and/or user device 202 are communicatively coupled to cloud 206 (when present), e.g. via the internet.

Cloud 206 includes a machine learning module 208. The machine learning module 208 is configured to perform aspects of the invention as described in more detail later in this specification. Machine learning module 208 can be selected according to the complexity of the sensor data that is provided to the module as input. Module 208 preferably implements is a deep learning algorithm, particularly in the case where sensor data of types B), C) and/or D) is suppled as input to module 208. The invention is however not limited to this and other machine learning algorithms can be used in place of deep learning.

One or more applications ('apps') can be installed on user device 202 and/or device 100 using a known installation technique. The one or more applications may include a scheduling application and/or a hair growth management device companion application. These may be provided as separate applications or as a single application offering both the scheduling and feedback functionality. The following provides a description of the functionality of these applications.

First a description of the companion application is provided. The companion application can be executed by user device 202 and/or device 100. A main function of the companion application is to provide feedback on a usage session based on data gathered during the usage session by the sensor(s) that are part of device 100. The feedback provided by the companion application includes a skin coverage. The skin coverage can be displayed using a graphical user interface that is displayed on display 204. The graphical user interface can include text and/or images. A percentage value may be displayed in text form indicating the skin coverage achieved in a given usage session. Additionally or alternatively a graphical representation of the skin coverage can be displayed, such as a shape having a fraction corresponding to the skin coverage achieved shaded and/or coloured. Alternative forms for the graphical user interface element will be apparent to a skilled person having the benefit of the present disclosure.

Other information relating to the usage session can be provided on the graphical user interface in addition to the skin coverage, such as any one or more of: an indication of the body part(s) that was/were exposed to light during the usage session; a total time of the usage session; an indication of the head(s) that was/were used during the usage session; textual feedback relating to the usage session such as a motivational message and/or a suggestion for increasing the skin coverage in a future usage session (e.g. 'move the device more slowly'). This list is not exhaustive and other information can additionally or alternatively be displayed.

In addition to data from the sensor(s) of device 100, the companion application can gather other information relating to the usage session. This information may be used to improve or supplement the skin coverage estimate. This information can include any one or more of:
- A skin tone of the skin that device 100 is applied to during the usage session. Skin tone information can be manually entered by a user, e.g. by comparing the skin to a skin tone swatch that may be displayed on display 204 of user device 202. Alternatively, if device 100 includes a skin tone sensor, then data gathered by this skin tone sensor can be provided to the companion application to allow an automatic determination of skin tone to be made. Techniques for using skin tone sensors to determine skin tone are known.
- A hair colour of the hair present on the skin that device 100 is applied to during the usage session. Hair colour information can be provided in the same manner as the skin tone, i.e. manually or automatically. A hair colour sensor can be provided for automatically detecting hair colour. Hair colour sensors are known.
- A hair density of the hair present on the skin that device 100 is applied to during the usage session. Hair density information can be provided by displaying two or more images representing different hair density, where each image corresponds to a different hair density. The user can select the image that corresponds most closely to the hair density of the skin that device 100 is applied to during the usage session. Alternatively, hair density may be automatically detected.
- An identifier of the body part(s) that device 100 is to be applied to during the usage session. This information can be manually entered by the user, or a body part sensor (not shown) may be used to automatically detect which body part(s) device 100 is proximate during the usage session.
- An identifier of the head(s) 104 of device 100 that were used / are to be used during the usage session. This information can be manually entered by the user, or a device head sensor (not shown) may be used to automatically detect which head(s) 104 is/are attached to housing 102 during the usage session. The companion application may also provide a recommendation for which head(s) should be used during the usage session based on the body part(s) that device 100 is to be used on. The recommendation could be text indicating which head should be used and/or an image of the head that should be used. The companion application may in this case warn the user if a suboptimal head is detected as being attached to device 100, e.g. by a textual warning displayed on display 204 and/or a graphic such as a warning sign. The warning may also suggest an optimal head for use during the next usage session of device 100.
- Information specific to the person that the device is to be used on in the usage session, such as: gender, name or some other identifier like an email address, username, phone number, etc., one or more goals of said person (e.g. a reduction in hair density by 50%, total hair removal, etc.), feedback from said person relating to previous usage sessions (e.g. satisfied, unsatisfied), etc. This information can be used to create and maintain a profile that stores information like the skin coverage achieved for each usage session of that person.
- A pulse energy or fluence as applied to the skin during the usage session, either as a cumulative value or on a 'per-pulse' basis. This information could be manually input but is preferably obtained automatically by device 100 by determining the pulse energy or fluence set during the usage session.
- Physiological information relating to the person that the device is to be used on in the usage session, such as height and/or weight. This information could be used as an input in the estimation of the skin coverage alongside the sensor data discussed above to further enhance the accuracy of the skin coverage estimate.

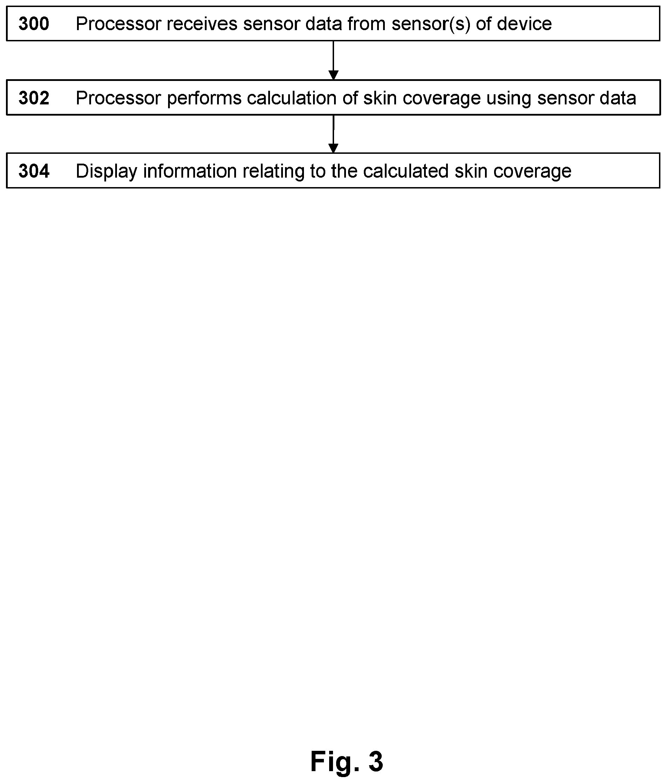

The companion application can be configured to monitor the usage of device 100 in a usage session according to the method of Fig. 3.

In step 300, a processor receives sensor data from one or more sensors of device 100, e.g. the sensors discussed above under A, B, C and/or D. The processor can be part of system 200. In some embodiments where processing complexity is on the lower side (e.g. embodiments that do not make use of machine learning), the processor is part of user device 202 or device 100. Conversely, in other embodiments where processing complexity is on the higher side (e.g. embodiments that make use of machine learning), the processor is part of cloud 206. The selection of processing resources in this way advantageously enables real-time or near real-time skin coverage estimates to be provided during the usage session, if desired.

The sensor data can be received by the processor as it is being gathered by device 100, i.e. while the usage session is ongoing. Alternatively the sensor data can be received by the processor after the usage session is complete.

The sensor data can be transmitted by a transmitter or transceiver of device 100 to the processor, e.g. a Bluetooth or WiFi transmitter / transceiver. In the case where the processor is in cloud 206, user device 202 may act as an intermediate device to route the sensor data to cloud 206 or device 100 can alternatively send the sensor data directly to cloud 206. As mentioned above, device 100 can be configured to transmit the sensor data continuously or quasi-continuously to another electronic device as a data stream.

In step 302, the processor performs a calculation of the skin coverage achieved in the usage session using the sensor data. In one embodiment where the sensor data comprises a flash count, the processor can be a processor of user device 202. In this embodiment a rules-based algorithm can be used by user device 202 to calculate a skin coverage based on flash count. This embodiment requires relatively little processing resource to calculate the skin coverage, hence user device 202 can handle the calculation itself. The user device 202 can thus perform the necessary calculations in or near real time, enabling a graphical user interface that shows progress of the usage session in or near real time to be displayed on display 206 as the usage session progresses. The graphical user interface could be a progress bar, for example. The progress bar or equivalent could be displayed at the end of a usage session, i.e. after the usage session is completed, or the progress bar or equivalent could be displayed and updated whilst the usage session is ongoing.

In another embodiment where the sensor data comprises at least accelerometer data, the processor is part of machine learning module 208 in cloud 206. In this case, device 100 may stream the sensor data directly to cloud 206 rather than introducing delays by sending the data via user device 202. The output of machine learning module 208, i.e. the estimated skin coverage, can be transmitted to user device 202 from cloud 206.

Machine learning module 208 can take the accelerometer data as input and output an estimated skin coverage. Any of the other sensor data types discussed earlier in this specification under A, B, C and/or D can additionally or alternatively be provided as input to machine learning module 208.

In a preferred form of this embodiment, machine learning module 208 is supplemented by a pre-processing rule-based algorithm that works together with machine learning module 208 to provide a skin coverage. In particular, some sensor data such as flash count and/or skin contact sensor data can be input into the rule-based algorithm and the output of the rule-based algorithm can be input into machine learning module 208 alongside other sensor data, e.g. accelerometer and/or gyroscope data. This can increase the accuracy of the skin coverage estimate provided by machine learning module 208.

A post-processing rule-based algorithm can be used in addition to the pre-processing rule-based algorithm, or as an alternative to the pre-processing rule-based algorithm. The post-processing rule-based algorithm can perform one or more so-called 'sanity checks' on the output from machine learning module 208. The sanity check(s) ensure that the output of machine learning module 208 makes sense, e.g. excluding a negative skin coverage value, or a value above 100%.

It will be appreciated that a plurality of post-processing rule-based algorithms and/or a plurality of pre-processing rule-based algorithms can be used.

In step 304, user device 202 displays on display 204 information relating to the calculated skin coverage, and/or this information is displayed on a display of device 100. The skin coverage information can be displayed in the manner discussed above, e.g. as a percentage, progress bar, etc. At least the skin coverage is displayed, e.g. as a numerical percentage and/or equivalent graphical representation. Other information as discussed above may additionally be displayed, e.g. a percentage of the target body part covered during the usage session; a usage session rating and/or a suggestion as to how a skin coverage of a subsequent usage session can be increased relative to the calculated skin coverage. The suggestion may be in text form, e.g. an operator instruction such as 'move the device more slowly over the skin', 'trigger more flashes', 'use a different head in the next usage session', etc. Information relating to the operation of device 100 may additionally or alternatively be displayed, e.g. a suggested energy level setting for a subsequent usage session.

The skin coverage value advantageously allows the user to gain immediate understanding of the effectiveness of a given usage of device 100. This is because it is generally appreciated by users that greater skin coverage leads to a more effective usage session. The skin coverage value is provided in a user-friendly manner as all the user need do is use device 100 and the calculation is taken care of automatically. Moreover, a single value - the skin coverage - is easy for an untrained user to understand such that they can immediately gain an objective perspective on the effectiveness of the usage session of device 100 based on this single, easy to understand parameter.

The skin coverage value can additionally be used to make predictions about the likely skin coverage in future usage session(s), potentially leading to the ability to predict the likely overall efficacy of a usage plan that comprises multiple usage sessions. (Usage plans are discussed later in this specification in more detail). This could involve converting the actual and/or predicted skin coverage into hair growth management efficacy, e.g. by using clinical data or other such efficacy data that relates skin coverage to hair growth management efficacy.

In embodiments where machine learning is made use of, the processor (e.g. a cloud-based processor that may be part of machine learning module 208) can be configured to store both the sensor data and the calculated skin coverage in a training dataset. This training dataset can be used by the processor to train a machine learning model to calculate a skin coverage.

Alternatively, in the case where a trained machine learning model already exists, the training dataset can be used to improve the trained machine learning model to arrive at a more accurate machine learning model. The trained or improved machine learning model can then be used by the processor to calculate a skin coverage for a subsequent usage session of device 100. The training or retraining can take place periodically (e.g. once a day, once a week, once a month) or the (re)training can be a continuous process.

It will be appreciated that configuring the processor in this way advantageously tends to increase the accuracy of the skin coverage calculation provided by machine learning module 208. This is because over time the training dataset grows, with a tendency to increase the effectiveness of the training process. Moreover, the training dataset can be supplemented by data from many different users, leading to a diverse and representative sample set for training.

In a second aspect the invention provides a scheduling application that is able to dynamically adjust a usage plan comprising one or more usage sessions of a light-emitting hair growth management device based on information relating to the hair density of a target body part or parts. This information can be provided as feedback received from a user on the level of hair growth management achieved in a given usage session. Alternatively, the level of hair growth management achieved in a given usage session can be automatically estimated. Further information on the second aspect is provide directly below. The second aspect can be combined with the first aspect, i.e. both the first and second aspects of the invention can be provided by a single application or separate companion and scheduling applications. The scheduling application can be executed by user device 202 and/or device 100.

The scheduling application of the second aspect functions to provide a mechanism for dynamically scheduling usage sessions. The scheduling application has a graphical user interface that includes a scheduling-type user interface that allows events to be scheduled. This might be, for example, a calendar of the type known per se in the art. The following description refers to a 'calendar application' and 'calendar' but this should be understood as one particular example of a scheduling application such that the invention is not limited to calendars and calendar applications in particular.

In the case of a calendar application, the scheduling user interface can include a calendar that indicates the day, week or month currently being shown. In week or month view, the calendar typically shows days in one or more rows, where each day is labelled by its corresponding date. A visual indicator such as a shape, shaded region, etc. may be present showing the current day.

Day(s) that currently have a usage session scheduled can be indicated in some manner, e.g. by showing their label in bold, a different colour, and/or with some visual indicator like a dot displayed proximate the label of the relevant day(s).

The user can interact with the calendar application via a user input component of user device 202 or device 100. The user can select a particular date and in response the calendar application provides more information about the selected date, e.g. in an expanded view. The information can include whether a usage session is scheduled for the selected date. Details relating to the usage session, e.g. target body part(s), can be provided if a usage session is scheduled.

Scheduled usage sessions can be part of a usage plan, this being understood as referring to multiple usage sessions that are grouped together. A usage plan may focus on a particular body part and may have a particular objective associated with it. A usage plan duration can be defined, this being the total time that it will take to complete the plan (e.g. 1 month, 2 months). The dates on which individual usage sessions of the usage plan are scheduled to take place can be shown as part of the usage plan.

Some exemplary usage plans are set out in Table A directly below. It should be appreciated that the information shown in Table A can be captured and stored by user device 202 as a data structure, e.g. in an XML format or JSON format data structure.

**Table A - exemplary usage plans**

| **Plan ID** | **Body part(s)** | **Objective** | **Start date** | **Usage count** | **Next usage date** | **Duration** |
|---|---|---|---|---|---|---|
| Plan 1 | Armpits | Temporary hair removal | 1 Jan 2022 | 3 | 1 Feb 2022 | 10 |
| Plan 2 | Face | Reduce visible hair | 15 Jan 2022 | 5 | 22 Feb 2022 | 15 |

The parameters shown in Table A are discussed in detail directly below.

Plan ID is a unique identifier assigned to the usage plan. This is typically a string of characters, e.g. 'Plan 1' as above. The plan ID can be automatically assigned by the user device as part of the creation of a new program. It may be possible for the user to edit or specify a plan ID.

Body part(s) is a parameter (e.g. a string) that identifies the body part or parts that the usage plan relates to. This information can be provided by the user during the creation of the usage plan. The user may select one or more body parts on an image of a body, for example. Alternatively, body part selection can be performed using a dropdown list of predefined items, a free text field with linked search functionality, etc.

The user may provide an objective for the usage plan and this can be stored as text (a string). This objective describes the outcome that the user wishes to achieve at the end of the usage plan. The objective can be selected from a predefined set of objectives, e.g. temporary hair removal, reduction of visible hair, slowing of hair growth, etc. Allowing the user to define an objective can advantageously assist with monitoring of the progress of the usage plan.

A start date of the usage plan can be set. The user can set the start date using a date picker user interface element, for example. The start date indicates the date on which the first usage session of the plan is scheduled to take place. This may be the date the usage plan is created, or a date in the future.

A usage count can be maintained by user device 202. The usage count is a number indicating the total number of usage sessions within the usage plan that have taken place.

A next usage date can be provided by user device 202. This indicates the date on which the next usage session in the usage plan is scheduled for. The next usage date can include just the next session (i.e. one date), or it can include all remaining usage session dates, i.e. a plurality of dates. This information can be displayed graphically to the user on the calendar graphical user interface element discussed above.

A duration of the usage plan can be set. The duration can be defined in terms of the total number of usage sessions that are within the usage plan, or it can be defined in terms of a time that it is expected to complete the usage plan (e.g. x days, weeks, months, etc.)

If calculated, a skin coverage value as discussed earlier in relation to the first aspect of the invention can be automatically added to calendar entries for usage sessions that have already taken place. The skin coverage can be viewable by the user when the date corresponding to the usage session is selected.

It will be appreciated that Table A is purely exemplary and that other information can additionally or alternatively be included in the data structure that represents the usage plan. Such information will be apparent to a skilled person having the benefit of the present disclosure and given the specifics of the situation at hand.

Any one or more of the parameters discussed above can be viewable by the user in a suitable graphical user interface. For example, user selection of a particular usage plan can cause user device 202 or device 100 to display a graphical user interface that shows the body part(s) related to the usage plan, a schedule indicating one or more upcoming usage sessions (e.g. by date, day, time, etc). An estimated completion date for the usage plan can be shown on the graphical user interface.

User device 202 and/or device 100 can be configured to provide reminders relating to a usage session based on the usage plan, e.g. using the 'next usage date' parameter. The reminder can be generated by user device 202 and/or device 100 in the form of an audio reminder such as an alarm, a visual reminder such as a notification message, or a combination of both audio and visual reminders. This list is not exhaustive and other reminder techniques can be used in addition or instead of the techniques listed here.

The user can configure the reminders that are generated using an appropriate graphical user interface element, e.g. setting a reminder type (audio/visual/both/other), setting a number of reminders that should be provided in advance of the usage session and/or setting a time period before the usage session that the reminder should be generated (e.g. 1 day before the usage session is scheduled, 1 hour before the usage session is scheduled, 10 minutes before the usage session is scheduled, etc.)

The calendar application may also provide a progress checker user interface that allows the user to check the progress of a given usage plan. The progress checker user interface can indicate the fraction of the usage plan that is complete, e.g. as a percentage or as a progress bar. As and when the user completes another usage session, the progress checker user interface is updated to show that this has occurred. In addition to the completed fraction, text and/or an image may be included on the progress checker user interface to enable the user to receive feedback about the current status of the usage plan. Text could include a motivational message, a comment about changes to their skin and/or hair density that the user might expect to see at the relevant point in the usage plan, and the like. An image could indicate the expected progress, e.g. a graphic of a strand of hair that gets progressively more transparent as the user progresses through the usage plan in order to represent hair reduction.

It will be appreciated that a usage plan can be defined in a computer-readable format, e.g. as a data structure of the type discussed above. Usage plans can thus be defined by a manufacturer or retailer of device 100 and downloaded to user device 202 and/or device 100 ready for use.

The usage plan as described above is a dynamic entity. That is, the usage plan can change after one or more usage sessions of the plan have been carried out. In particular, the date of one or more of the usage sessions yet to be carried out can be rescheduled after the usage plan has been started. Additionally or alternatively, one or more of the usage sessions yet to be carried out can be skipped (cancelled), and/or one or more additional usage sessions can be added to the usage plan.

Adjustments to the usage plan are made based on the effect that the usage session(s) that have already taken place have had on the hair of the person that device 100 is being applied to. For example, if it is found that hair growth reduction is occurring more quickly than initially expected, the usage plan can be adjusted so that there are larger amounts of time between adjacent future scheduled usage sessions and/or one or more scheduled usage sessions that have not yet taken place may be cancelled. Alternatively, if it is found that hair growth reduction is occurring more slowly than initially expected, or no reduction has been found and in fact hair (re)growth is occurring, the usage plan can be adjusted so that there are smaller amounts of time between adjacent future scheduled usage sessions and/or one or additional usage sessions may be added to the usage plan.

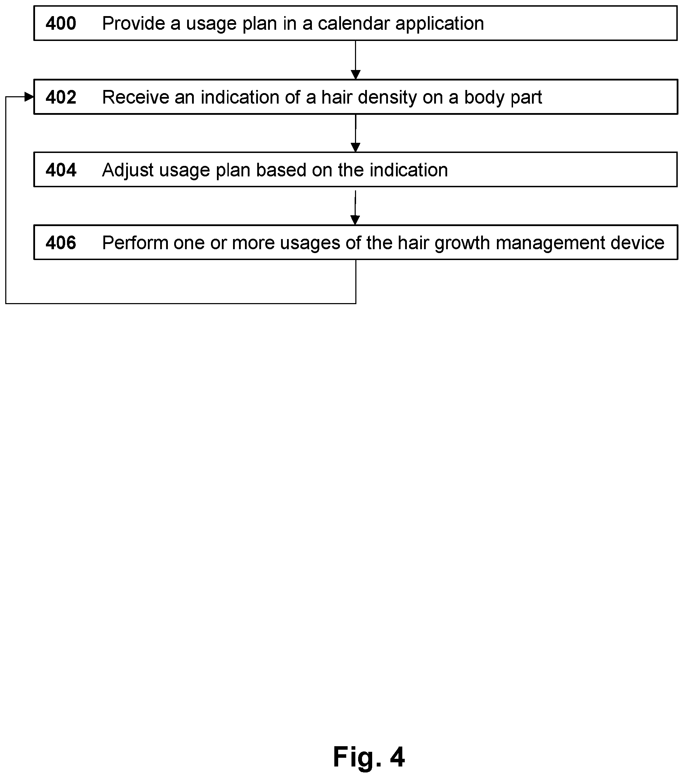

Adjustment to the usage plan can be performed automatically according to the method shown in Fig. 4. The adjustment can be performed using a machine learning algorithm, preferably a recurrent neural network (RNN) or a HMM. The adjustment can be carried out in the cloud, e.g. by machine learning module 208 or by user device 202. Preferably the location at which the processing is performed (user device 202 or cloud 206) is selected based upon the complexity of the machine learning algorithm, with cloud 206 being selected for more complex algorithms and user device 202 being selected for less complex algorithms.

Adjustments to the usage plan can also be made manually by the user. For example, a person may be unexpectedly unavailable for a particular usage session, causing the usage session to be manually skipped or rescheduled. The calendar application can include a graphical user interface that allows usage sessions within the usage plan to be skipped or rescheduled. The calendar application can also automatically detect usage sessions that were skipped, e.g. based on the lack of user input relating to such a usage session.

In a preferred embodiment, the adjustment is performed by a combination of a rule-based algorithm and a machine learning algorithm. The rule-based algorithm can be a pre-processing rule-based algorithm and/or a post-processing rule-based algorithm. The pre-processing rule-based algorithm can be configured to receive information relating to scheduling, e.g. usage session(s) that have been skipped, manually rescheduled or missed. This may be used by the pre-processing rule-based algorithm to calculate one or more parameters that can be fed as input into the machine learning model along with a hair density indication as discussed below.

A post-processing rule-based algorithm can be used in addition to the pre-processing rule-based algorithm, or as an alternative to the pre-processing rule-based algorithm. The post-processing rule-based algorithm can perform one or more sanity checks on the output from the machine learning model. The sanity check(s) ensure that the output of the machine learning model makes sense, e.g. a value output by the machine learning model is a valid date that is not in the past. The post-processing rule-based algorithm may include safety-based elements, e.g. preventing the time between adjacent usage sessions being less than a threshold 'safe limit' value. Other post-processing rules could be based on user convenience, e.g. preventing the usage plan from being changed excessively often which could annoy or otherwise inconvenience a user.

It will be appreciated that a plurality of post-processing rule-based algorithms and/or a plurality of pre-processing rule-based algorithms can be used.

Turning now to Fig. 4, the adjustment process is shown. The following description focusses on user device 202 performing various aspects of the steps of Fig. 4, but it should be appreciated that these operations can alternatively be performed by device 100.

In in step 400, user device 202 provides a usage plan in a calendar application of the type discussed above. The usage plan comprises one or more planned usage events. The or each planned usage event has a corresponding date associated with it. This is also as discussed above. The usage plan could be downloaded to user device 202 from a manufacturer or retailer computer, for example. Alternatively, the usage plan could be created by user device 202 by asking the user a series of questions, e.g. which body part(s) the usage plan should relate to, the skin tone and/or hair colour of the person that device 100 is to be used on, the objective that the person that device 100 has regarding the use of device 100, etc. A 'default' or 'initial' usage plan can be created based on this information, with this usage plan being subject to modification as described below in connection with Fig. 3.

In step 402, user device 202 obtains an indication of a hair density of hair present on a body part associated with the usage plan. This indication can be obtained manually or automatically.

In the manual case, user device 202 receives user input via a user interface of usage device 204. The user input includes an indication of a hair density on the body part(s) that the usage plan is associated with.

In order to assist the user in providing an objective indication of hair density, user device 202 can display a hair density graphical user interface element on display 206. The hair density graphical user interface element comprises at least two images representing different hair density, where each image corresponds to a different hair density. The user is then able to inspect the skin that device 100 is to be used on and compare the hair density observed by eye with the images presented on the user interface.

Each image could be a photograph of skin having a particular hair density. Alternatively each image could be a graphical representation of skin having a particular hair density, e.g. a drawing, sketch, animation, etc.

The user can select the image that most closely resembles the current hair density for the skin that device 100 is being used on. User device 202 can register this selection and thus receive an indication of the hair density on the body part that relates to the usage plan.

One or more images of the skin that the device is to be used on may also be displayed to allow a 'side by side' comparison with the images displayed in the hair density graphical user interface element . This may be more intuitive for the user. This/these image(s) may be captured by a camera or other such imaging module of user device 202.

In the case where the usage plan relates to multiple body parts, step 402 can be repeated for each body part and the user's selection recorded separately for each body part.

It will be appreciated that one or more uses of device 100 can take place between steps 400 and 402 such that the hair density indicated in step 402 may not be the starting hair density of the body part that device 100 is used on.

In the case where the indication of the hair density is obtained automatically, one or more images of the skin that the device is to be used on are obtained, e.g. by a camera of user device 202. This/these image(s) are processed to enable a hair density to be estimated. The image processing can be carried out by an image processing algorithm, e.g. a machine learning algorithm that has been trained to detect pixels of an image that are likely to correspond to a hair. The detection of hairs in the image can allow a hair density to be estimated.

In step 404, the usage plan is adjusted based on the indication obtained in step 402. Adjusting includes altering a data of at least one of the planned usage events that form part of the usage plan. The date may be rescheduled by bringing it forward (i.e. making it closer to the current date) or pushing it back (i.e. making it further away from the current date). More than one of the planned usage events can be rescheduled at once, e.g. all planned usage events moved forwards or backwards by one day, two days, one week, etc. Alternatively, the planned usage events may be rescheduled so that there is a particular amount of time between adjacent events, e.g. one week.

In addition to date adjustments, the usage plan can be adjusted in step 404 by adding one or more additional planned usage events and/or skipping/deleting one or more existing planned usage events.

The usage plan can be adjusted by providing the indication received in step 402 and the usage plan itself to a machine learning module. The machine learning module is trained to adjust a usage plan based on the indication received from the user. The machine learning module outputs the adjusted usage plan. The machine learning module may be module 208 shown in Fig. 2, for example. The machine learning module may make use of a RNN or preferably a HMM, although the invention is not limited in this regard and other types of machine learning algorithm can be used instead.

The calendar in the calendar application can be automatically updated by user device 202 to take account of the adjustments to the usage plan made in step 404. Any alarms, reminders, etc. that user device 202 has set can also be automatically adjusted. A notification may be displayed on display 206 to enable the user to confirm that the adjustment has been made.

Following step 404, in step 406 one or more subsequent usages of device 100 can be made according to the adjusted usage plan. As shown in Fig. 4, it is possible to repeat steps 402 and 404 after a subsequent usage of device 100 to further adjust the usage plan.

In this way the usage plan is made dynamic as it is responsive to the actual effects that device 100 has on the hair of the target body part(s). This can advantageously lead to improved user experience and/or improved user satisfaction.

The dynamic adjustment of the usage plan can take into account safety-related factors. For example, safety rules may be built into the logic that adjusts the usage plan to prevent the usage plan from suggesting practices that are considered unsafe. For example, the usage plan may be prevented by one or more safety rules from being adjusted in a way that places adjacent usage sessions closer together in time than is recommended. This prevents the usage plan from suggesting a usage pattern that may cause harm to the skin of the person device 100 is to be used on.

As in the case of the skin coverage parameter, the processor (e.g. a cloud-based processor that may be part of machine learning module 208) can be configured to store the usage plan and hair density indication in a training dataset. This training dataset can be used by the processor to train a machine learning model to determine recommended adjustments to the usage plan. Alternatively, in the case where a trained machine learning model already exists, the training dataset can be used to improve the trained machine learning model to arrive at a more accurate machine learning model. The trained or improved machine learning model can then be used by the processor to calculate adjustments to a usage plan for a subsequent iteration of at least steps 402 and 404 of Fig. 4.

It will be appreciated that configuring the processor in this way advantageously tends to increase the suitability of the adjusted usage plan provided by machine learning module 208. This is because over time the training dataset grows, with a tendency to increase the effectiveness of the training process. Moreover, the training dataset can be supplemented by data from many different users, leading to a diverse and representative sample set for training.

It will be appreciated that the operations described herein can be performed by a processor according to computer-readable instructions stored on a computer-readable medium. The computer-readable medium may be non-transitory. One or more computer-readable media storing such instructions therefore also form part of the present invention.

In addition to the embodiments described above, the following clauses set out further embodiments of the invention.

Clause 1: A computer-implemented method for adjusting a usage plan for a light-emitting hair growth management device, the method comprising: providing, in a scheduling application of an electronic device, a usage plan comprising one or more planned usage events, the or each planned usage event having a corresponding date associated with said or each planned usage event; obtaining, by the electronic device, an indication of a hair density of hair present on a body part associated with the usage plan; and adjusting, by the electronic device, a date of at least one of the one or more planned usage events based on the indication.

Clause 2: The computer-implemented method of clause 1, wherein adjusting, by the electronic device, a date of at least one of the one or more planned usage events based on the user input comprises: providing the indication and the usage plan to a machine learning module configured to adjust the usage plan based on the user input; and receiving an adjusted usage plan as an output of the machine learning model.

Clause 3: The computer-implemented method of clause 1 or clause 2, further comprising: displaying, on a display of the electronic device, a hair density graphical user interface element comprising at least two images representing different hair density, each said image corresponding to a different hair density; wherein the step of obtaining, by the electronic device, an indication of the hair density of hair present on a body part comprises receiving a user selection of one of the at least two images representing different hair density.

Clause 4: The computer-implemented method of clause 1 or clause 2, wherein the step of obtaining, by the electronic device, an indication of the hair density of hair present on a body part comprises: obtaining one or more images, each of the one or more images including at least a part of the body part; and automatically processing the one or more images to estimate the hair density.

Clause 5: A non-transitory computer-readable medium storing instructions thereon which, when executed by a processor of an electronic device, cause the electronic device to: provide, in a scheduling application of an electronic device, a usage plan comprising one or more planned usage events, the or each planned usage event having a corresponding date associated with said or each planned usage event; obtain an indication of a hair density of hair present on a body part associated with the usage plan; and adjust, by the electronic device, a date of at least one of the one or more planned usage events based on the indication.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A computer-implemented method for monitoring the usage of a light-emitting hair growth management device in a usage session of the hair growth management device, the method comprising:
receiving, by a processor, sensor data from one or more sensors of the hair growth management device, the sensor data gathered during the usage session;
performing, by the processor, a calculation of a skin coverage achieved in the usage session using the sensor data; and
displaying, on a display of a user device or on a display of the hair growth management device, information relating to the calculated skin coverage.

2. The computer-implemented method of claim 1, wherein the processor is a processor of the user device or the hair growth management device and the sensor data comprises at least a number of flashes of light emitted by the hair growth management device during the usage session.

3. The computer-implemented method of claim 1 or claim 2, wherein the displaying of the information relating to the calculated skin coverage comprises displaying a graphical user interface indicating a fraction of a target body part covered in the usage session, the method further comprising:
updating, by the processor, the displaying of the graphical user interface during the usage session or after the usage session is complete.

4. The computer-implemented method of one of claims 1 to 3, wherein at least a portion of the processor is a cloud-based processor and the calculation of the skin coverage achieved is performed using a machine learning algorithm.

5. The computer-implemented method of one of claims 1 to 4, wherein the sensor data comprises one or more of:
accelerometer data of an accelerometer sensor of the hair growth management device, the accelerometer data collected during the usage session;
rotational data of a gyroscope of the hair growth management device, the rotational data collected during the usage session;
a count of a number of flashes of light emitted by the hair growth management device during the usage session; and/or
skin contact sensor data of a skin contact sensor gathered during the usage session.

6. The computer-implemented method of one of claims 1 to 5, further comprising:
transmitting, by a transmitter or transceiver of the hair growth management device, the sensor data to the processor.

7. The computer-implemented method of one of claims 1 to 5, the method further comprising:
transmitting, by a transmitter or transceiver of the hair growth management device, the sensor data to the user device; and
transmitting, by a transmitter or transceiver of the user device, the sensor data to the processor.

8. The computer-implemented method of claim 6 or claim 7, wherein the sensor data is transmitted continuously or quasi-continuously during the usage session as a data stream.

9. The computer-implemented method of any one of claims 1 to 7, further comprising:
storing, by the processor, the sensor data and the calculated skin coverage in a training dataset;
training, by the processor, a machine learning model using the training dataset to produce a trained machine learning model or to improve an existing machine learning model; and
using, by the processor, the trained or improved machine learning model to calculate a skin coverage for a subsequent usage session of the hair growth management device.

10. The computer-implemented method of any preceding claims, wherein the displaying of the information relating to the calculated skin coverage includes any one or more of:
displaying a percentage of a target body part covered during the usage session;
displaying a usage session rating; and/or
displaying a suggestion as to how a skin coverage of a subsequent usage session can be increased relative to the calculated skin coverage.

11. The computer-implemented method of any preceding claim, wherein the hair growth management device comprises a head sensor configured to detect a type of a head currently removably attached to the hair growth management device, the method further comprising:
detecting a type of a head currently attached to the hair growth management device based on data from the head sensor;
determining whether the currently attached head is an optimal head for a next usage session of the hair growth management device; and
in the case where the currently attached head is suboptimal, displaying on the display of the user device a notification indicating that the currently attached head is suboptimal for a next usage session of the hair growth management device.

12. The computer-implemented method of claim 11, wherein the notification also includes a suggestion of an optimal head for a next usage session of the hair growth management device.

13. The computer-implemented method of any preceding claim, further comprising:
displaying, on the display of the user device, a suggestion of an energy level setting for a next usage session of the hair growth management device.

14. A computer program which, when executed by one or more processors, causes the one or more processors to perform the method of any one of claims 1 to 13.

15. A hair growth management system comprising a hair growth management device, a processor and a display, the hair growth management system configured to perform the method of any one of claims 1 to 13.
